# EUROPÄISCHE PATENTSCHRIFT

(11) **EP 0 951 880 B1**
(45) Veröffentlichungstag und Bekanntmachung des Hinweises auf die Patenterteilung: **29.10.2003**
(21) Anmeldenummer: 99101207.1
(22) Anmeldetag: 22.01.1999
(51) Int. Cl.: A61F 2/52

(54) **Verfahren zum Aufbringen einer dauernd klebrig bleibenden Schicht auf die Innenseite einer Brustprothese**
Process for depositing a durably sticky layer upon the interior surface of a mammary prosthesis
Procédé pour déposer une couche durablement collante sur la surface intérieure d'une prothèse mammaire

(30) Priorität: 21.04.1998 DE 19817769
(43) Veröffentlichungstag der Anmeldung: 27.10.1999
(60) Teilanmeldung: 03014843.1
(73) Patentinhaber: AMOENA Medizin-Orthopädie-Technik GmbH, D-83064 Raubling (DE)
(72) Erfinder: Reitmaier, Paul, 83547 Babensham (DE); Esterer, Ulrike, 83352 Altenmarkt (DE); Stelter, Nils, 83126 Flintsbach (DE); Stuffer, Georg, 83126 Flintsbach (DE); Stuffer, Hans, 83131 Nussdorf (DE)
(74) Vertreter: Gossel, Hans K., Dipl.-Ing.

(56) Entgegenhaltungen:
- EP-A- 0 542 119
- DE-A- 2 737 321
- DE-A- 4 211 542
- DE-U- 9 107 507

## Beschreibung

Die Erfindung betrifft ein Verfahren zum Aufbringen einer dauernd klebrig bleibenden Schicht aus einer klebrig eingestellten additionsvernetzenden Zweikomponentensilikonkautschukmasse auf die Innenseite einer Brustprothese, bestehend aus einem schalenförmigen Körper aus einem weichelastischem Material, vorzugsweise aus einem Körper aus einer weich elastisch eingestellten additionsvernetzenden Zweikomponentensilikonkautschukmasse, der in Kunststoffolien eingeschweißt ist, die dessen Außenseite und dessen Innenseite abdecken, und der in einer Form unter Zuführung von Wärme ausgehärtet ist.

Aus EP 0 542 119 B1 sind Verfahren dieser Art bekannt, nach denen einmal eine klebrig eingestellte additionsvernetzende Zweikomponentensilikonkautschukmasse zur Bildung der dauernd klebrig bleibenden Schicht mit einer Tragfolie in eine Form eingebracht und in dieser ausgehärtet wird und anschließend die Tragfolie mit der die Prothese einhüllenden Folie auf ihrer Rückseite in ihrem Randbereich verklebt oder verschweißt wird und zum anderen eine klebrig eingestellte additionsvernetzende Zweikomponentensilikonkautschukmasse zur Bildung der dauernd klebrig bleibenden Schicht mit einer Tragfolie in eine Form eingebracht und in dieser ausgehärtet wird und auf die andere Seite der Tragfolie ein Schmelzkleber aufgebracht wird und anschließend die Tragfolie auf die die Rückseite des Körpers einhüllende Folie aufgebracht und während des Aushärtungsprozesses des Körpers durch Aufschmelzen und Erhärten des Schmelzklebers mit der Folie verbunden wird.

Aufgabe der Erfindung ist es, weitere Verfahren vorzuschlagen, nach denen sich in einfacher und vorteilhafter Weise dauernd klebrig bleibende Schichten mit Brustprothesen der eingangs angegebenen Art verbinden lassen.

Diese Aufgabe wird dadurch gelöst, daß die Klebeschicht unter Verbindung mit einer Tragfolie ausgehärtet und die Tragfolie mit der noch flach liegenden und längs eines umlaufenden Randes verschweißten späteren Prothesenhülle verklebt wird und daß sodann die Prothesenhülle mit dem additionsvernetzenden Zweikomponentensilikonkautschuk gefüllt und in einer Form ausgehärtet wird.

Die Klebeschicht wird in bekannter Weise separat in einer Form hergestellt, in der sie sich mit der Tragfolie verbindet.

Die Klebeschicht kann mit der Tragfolie auf eine Folie oder Folienbahn schon aufgebracht werden, bevor aus dieser die Prothesenhülle hergestellt wird.

Nach einer bevorzugten Ausführungsform ist vorgesehen, daß die Klebeschicht unmittelbar auf die Hülle oder die Bahn vor der Herstellung der Hülle aufgebracht wird, und zwar ohne Vermittlung über eine Trägerschicht.

Nach einer weiteren bevorzugten Ausführungsform ist vorgesehen, daß auf die die spätere Prothesenrückseite bildende Bahn vor der Herstellung der Hülle die Klebeschicht vollständig aufgebracht wird. Wird diese mit der Klebeschicht versehene spätere Prothesenrückseite bildenden Folie mit der die Prothesenvorderseite bildende Folie längs eines umlaufenden Randes verschweißt, drücken die Schweißwerkzeuge die Klebeschicht durch, so daß es zu einer guten Verschweißung der die flachliegende Hülle bildenden Folienbahnen kommt. Nach der Herstellung der Prothesen werden die dauernd klebrig bleibenden Schichten durch Trennfolien geschützt, die vor dem Anlegen der Prothesen abgezogen werden.

Ausführungsbeispiele der Erfindung werden nachstehend anhand der Zeichnung näher erläutert. In dieser zeigt
- Fig. 1: einen Querschnitt durch eine in einer nicht dargestellten Form liegende Brustprothese, die durch einen Formdeckel abgedeckt ist, der auf seiner der Prothese zugewandten Seite mit einem der aufzubringenden Klebeschicht entsprechenden Kanal versehen ist,
- Fig. 2: eine der Fig. 1 entsprechende Darstellung, bei der zwischen dem Formdeckel und der Prothesenrückseite ein flexibler Streifen mit einer Ausnehmung eingelegt ist, die mit dem Klebestreifen gefüllt ist, und
- Fig. 3: eine der Fig. 1 entsprechende Darstellung, bei der auf die Prothesenrückseite eine abziehbare Folie aufgebracht ist, zwischen der und der Prothesenrückseite ein der Form der Klebeschicht entsprechender Kanal eingebracht ist, der mit dem die Klebeschicht bildenden Zweikomponentensilikonkautschuk gefüllt ist.

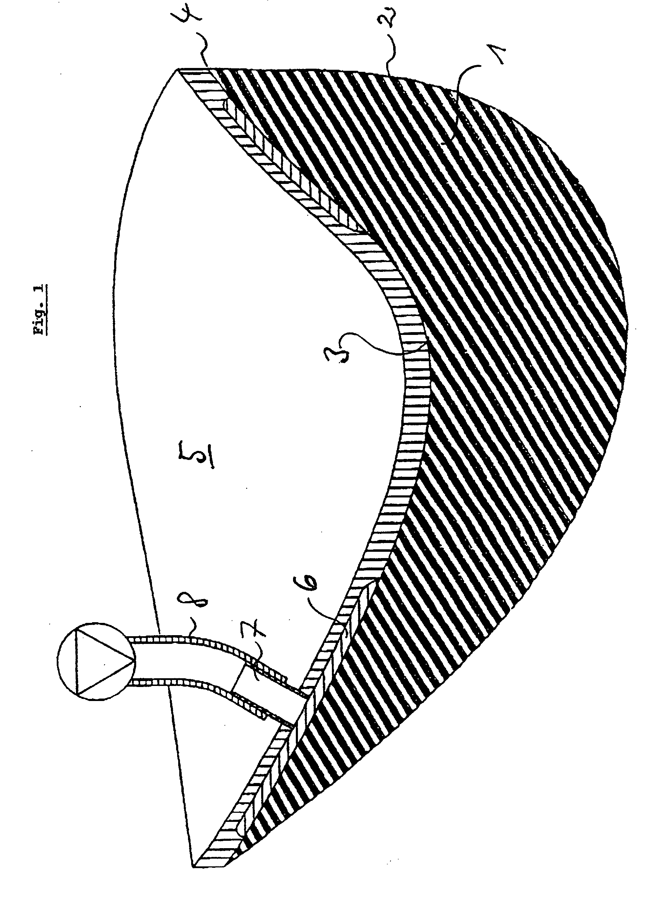

Nach dem Ausführungsbeispiel der Fig. 1 ist in eine nicht dargestellte Unterschale einer Form eine mit einem additionsvemetzenden Zweikomponentensilikonkautschuk 1 gefüllte Hülle eingelegt, die aus einer die Prothesenvorderseite abdeckenden Folie 2 und einer die Prothesenrückseite abdeckenden Folie 3 besteht, die längs ihres umlaufenden Randes 4 miteinander verschweißt sind. Die Prothesenhülle wird vor der Befüllung aus flach aufeinanderliegenden PU-Folien hergestellt. Der Zweikomponentensilikonkautschuk wird durch eine Randöffnung eingefüllt, so daß die Prothesenhülle aufgrund der Elastizität des Folienwerkstoffs die Form der späteren Brustprothese annimmt.

Der Formdeckel 5 weist auf seiner der Prothesenrückseite zugewandten Seite eine flache einen Kanal bildende Ausnehmung 6 auf, deren Form eine Negativform der auf die Prothesenrückseite aufzubringenden Klebeschicht ist. In den Kanal mündet ein in eine Bohrung des Formdeckels eingesetzter Stutzen 7, an den ein Schlauch 8 angeschlossen ist, über den in die rinnenartige Ausnehmung 6 nach dem Schließen des Formdeckels 5 der die Klebeschicht bildende Zweikomponentensilikonkautschuk über eine Pumpe eingefüllt wird.

Nach dem Schließen der Form und dem Einbringen des die Klebeschicht bildenden Zweikomponentensilikonkautschuks wird diese zusammen mit dem Prothesenkörper in üblicher Weise in einem Ofen ausgehärtet.

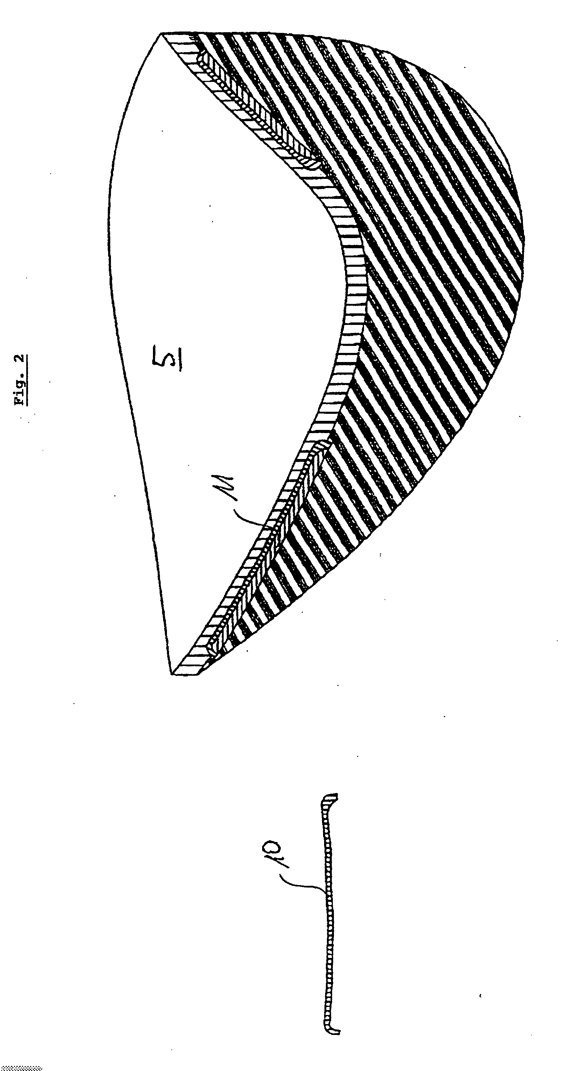

Bei dem Ausführungsbeispiel nach Fig. 2 wird zunächst in einer flachen Schale aus einem elastischen und/oder plastischen thermoplastischen Werkstoff mit einer rinnenförmigen Ausnehmung, die der Form der späteren Klebeschicht entspricht, in einem besonderen Herstellungsprozeß eine dauernd klebrig bleibende Schicht aus einer Zweikomponentensilikonkautschukmasse hergestellt. Die Schale 10 mit der in dieser ausgehärteten Klebeschicht 11 wird sodann in eine der Schalenform angepaßte Aussparung des Formdeckels 5 eingelegt und mit dem so vorbereiteten Formdeckel wird sodann die Form geschlossen. Die dauernd klebrig bleibende Schicht wird sodann nochmals zusammen mit dem Prothesenkörper in einem Ofen ausgehärtet, so daß sich die Klebeschicht mit der die Prothesenrückseite abdekkenden Folie verbindet. Nach dem Öffnen der Form wird die flache Schale 10 von der Klebeschicht abgezogen, so daß sie erneut verwendet werden kann. Die so hergestellte und mit der Prothesenrückseite verbundene Klebeschicht wird dann durch eine Trennfolie abgedeckt.

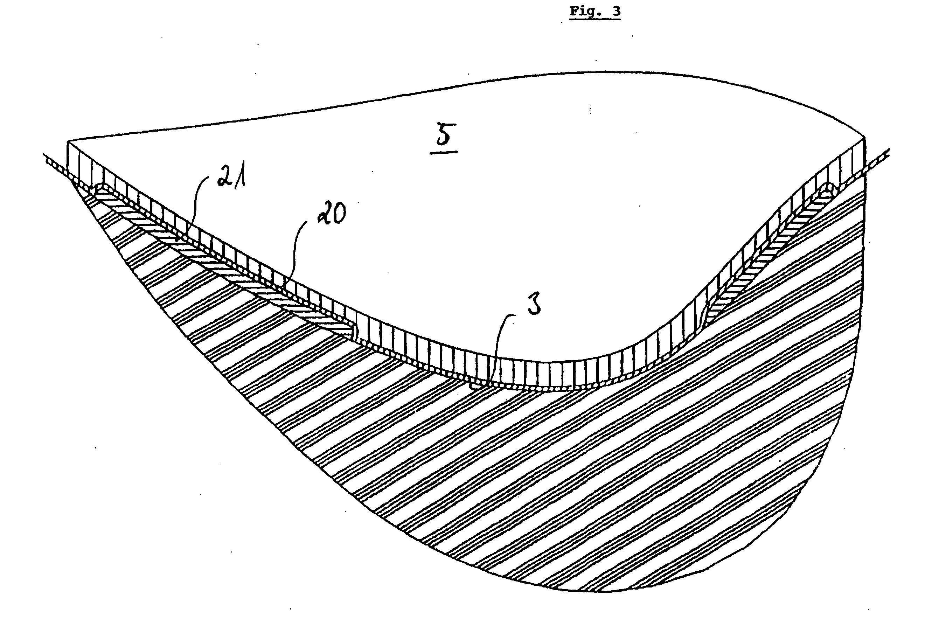

Bei dem Ausführungsbeispiel nach Fig. 3 wird auf die die Prothesenrückseite bildende Folie 3 eine abziehbare Trennfolie 20 aufgebracht, die mit der Folie 3 in der Weise verbunden ist, daß zwischen beiden ein Kanal 21 gebildet ist, der der Form der aufzubringenden Klebeschicht entspricht. Dieser Kanal 21 wird vor dem Schließen der Form mit dem Formdeckel 5 mit einer die Klebeschicht bildenden Zweikomponentensilikonkautschukmasse gefüllt. In den Formdeckel ist ein Kanal eingearbeitet, der der Form der Klebeschicht entspricht. Nachdem der Kanal mit der die Klebeschicht bildenden Zweikomponentensilikonkautschukmasse gefüllt ist, wird nach dem Schließen der Form diese mit dem Prothesenkörper ausgehärtet. Der Form kann sodann die Prothese entnommen werden, wobei die Klebeschicht durch die Trennfolie 21 geschützt ist, die von der Trägerin erst vor dem Anlegen abgezogen wird.

## Patentansprüche

1. Verfahren zum Aufbringen einer dauernd klebrig bleibenden Schicht aus einer klebrig eingestellten additionsvernetzenden Zweikomponentensilikonkautschukmasse auf die Innenseite einer Brustprothese, bestehend aus einem schalenförmigen Körper aus einem weichelastischem Material, vorzugsweise aus einem Körper aus einer weichelastisch eingestellten additionsvernetzenden Zweikomponentensilikonkautschukmasse, der in Kunststoffolien eingeschweißt ist, die dessen Außenseite und dessen Innenseite abdecken, und der in einer Form unter Zuführung von Wärme ausgehärtet ist,
**dadurch gekennzeichnet,**
**daß** die Klebeschicht unter Verbindung mit einer Tragfolie ausgehärtet und die Tragfolie mit der noch flachliegenden und längs eines umlaufenden Randes verschweißten späteren Prothesenhülle verklebt wird und daß sodann die Prothesenhülle mit dem additionsvernetzenden Zweikomponentensilikonkautschuk gefüllt und in einer Form ausgehärtet wird.

2. Verfahren nach Anspruch 1, **dadurch gekennzeichnet, daß** die Klebeschicht mit der Tragfolie auf eine Folie oder Folienbahn schon vor der Herstellung der Prothesenhülle aufgebracht wird.

3. Verfahren nach Anspruch 1, **dadurch gekennzeichnet, daß** die Klebeschicht unmittelbar auf die Hülle oder die Bahn vor der Herstellung der Hülle aufgebracht wird.

4. Verfahren nach Anspruch 1, **dadurch gekennzeichnet, daß** auf die die spätere Prothesenrückseite bildende Bahn vor Herstellung der Hülle die Klebeschicht vollflächig aufgebracht wird.

## Claims

1. Method for the application of a layer remaining permanently adhesive made from an adhesively formulated addition-crosslinking two-component silicone rubber mass to the inside of a breast prosthesis consisting of a shell-shaped body made from a flexible material, preferably of a body made from a flexibly formulated addition-crosslinking two-component silicone rubber mass, which body is welded in plastic films which cover its outside and its inside, and which is cured in a mould while heat is supplied,
**characterized**
**in that** the adhesive layer is cured with joining to a carrier film and the carrier film is adhered to the later prosthesis sheath, which is still lying flat and which is welded along a circumferential edge, and in that the prosthesis sheath is then filled with the addition-crosslinking two-component silicone rubber and is cured in a mould.

2. Method according to Claim 1, **characterized in that** the adhesive layer with the carrier film is applied to a film or film web even before the prosthesis sheath is produced.

3. Method according to Claim 1, **characterized in that** the adhesive layer is applied directly to the film or web before the sheath is produced.

4. Method according to Claim 1, **characterized in that** the adhesive layer is applied to the full area of the web which forms the later reverse of the prosthesis, before the sheath is produced.

## Revendications

1. Procédé pour déposer une couche durablement collante composée d'une masse de caoutchouc de silicone à deux composants réticulant par addition sur la surface intérieure d'une prothèse mammaire qui est constituée d'un corps en forme de coquille fait d'un matériau élastique et tendre, de préférence d'un corps fait d'une masse de caoutchouc de silicone à deux composants, réticulant par addition, ajustée pour être tendre et élastique, qui est soudé dans des feuilles de plastique couvrant sa surface extérieure et sa surface intérieure et durci dans un moule par l'introduction de chaleur,
**caractérisé en ce que**
la couche collante est durcie en jonction avec une feuille de support et, la feuille de support est encollée avec ce qui formera l'enveloppe prothétique, qui est encore positionnée à plat et soudée le long d'un bord périphérique et ensuite, l'enveloppe prothétique est remplie avec le caoutchouc de silicone à deux composants réticulant par addition et durcie dans un moule.

2. Procédé selon la revendication 1, **caractérisé en ce que** la couche collante est déposée sur une feuille ou une bande de feuilles avec la feuille de support, avant la réalisation de l'enveloppe prothétique.

3. Procédé selon la revendication 1, **caractérisé en ce que** la couche collante est déposée directement sur l'enveloppe ou la bande avant la réalisation de l'enveloppe.

4. Procédé selon revendication 1, **caractérisé en ce que** la couché collante est déposée sur toute la surface de la bande qui formera la future face arrière de la prothèse avant la réalisation de l'enveloppe.
